# EUROPEAN PATENT SPECIFICATION

(11) **EP 4 110 329 B1**
(45) Date of publication and mention of the grant of the patent: **22.07.2026**
(21) Application number: 21761300.9
(22) Date of filing: 24.02.2021
(51) Int. Cl.: A61K 31/407, A61P 37/00

(54) **KETOROLAC FOR USE IN THE TREATMENT OF CYTOKINE STORM AND CYTOKINE RELEASE SYNDROME**
KETOROLAC ZUR BEHANDLUNG CON ZYTOKINSTURM UND ZYTOKINFREISETZUNGSSYNDROM
KETOROLAC POUR LE TRAITEMENT D'UN CHOC CYTOKINIQUE ET D'UN SYNDROME DE LIBÉRATION DE CYTOKINES

(30) Priority: 24.02.2020 US 202062980652 P
(43) Date of publication of application: 04.01.2023
(73) Proprietor: Yinuoke Medicine Science Technology Company Ltd., Changchun, Jilin, 130012 (CN); Zhang, Lingbing, Davis, CA 95616 (US)
(72) Inventor: ZHANG, Lingbing, Davis, CA 95616 (US)
(74) Representative: J A Kemp LLP
(86) International application number: PCT/US2021/019462
(87) International publication number: WO 2021/173707

(56) References cited:
- WO-A1-2019/165240
- MX-A- 2013 005 378
- US-A- 5 382 591
- US-A1- 2019 175 649
- ANONYMOUS: "Ketorolac tromethamine tablets USP, 10 mg", DAILYMED - FDA, 1 June 2015 (2015-06-01), pages 1 - 21, XP093129556, Retrieved from the Internet <URL:https://dailymed.nlm.nih.gov/dailymed/fda/fdaDrugXsl.cfm?setid=1191f151-ab29-1f7e-e054-00144ff88e88&type=display>
- CHANG LING ET AL: "Cytokine storm: a therapeutic target for severe new coronavirus infection?", 6 February 2020 (2020-02-06), XP055813189, Retrieved from the Internet <URL:http://www.csi.org.cn/article/content/view?id=1006> [retrieved on 20210611]
- MI JIAN-QING ET AL: "Effective Treatment of Relapsed/Refractory Multiple Myeloma Including Extramedullary Involvement By BCMA-Specific Chimeric Antigen Receptor-Modified T Cells", BLOOD, AMERICAN SOCIETY OF HEMATOLOGY, US, vol. 130, 8 December 2017 (2017-12-08), pages 3115, XP086631768, ISSN: 0006-4971, DOI: 10.1182/BLOOD.V130.SUPPL_1.3115.3115
- PORTER DAVID ET AL: "Grading of cytokine release syndrome associated with the CAR T cell therapy tisagenlecleucel", vol. 11, no. 1, 1 December 2018 (2018-12-01), XP093102069, Retrieved from the Internet <URL:https://jhoonline.biomedcentral.com/counter/pdf/10.1186/s13045-018-0571-y.pdf> DOI: 10.1186/s13045-018-0571-y
- RUSSO ROBERTO ET AL: "Ketogal: A Derivative Ketorolac Molecule with Minor Ulcerogenic and Renal Toxicity", FRONTIERS IN PHARMACOLOGY, vol. 8, 6 November 2017 (2017-11-06), CH, XP093129842, ISSN: 1663-9812, DOI: 10.3389/fphar.2017.00757
- J. R. TISONCIK ET AL: "Into the Eye of the Cytokine Storm", MICROBIOLOGY AND MOLECULAR BIOLOGY REVIEWS, vol. 76, no. 1, 1 March 2012 (2012-03-01), pages 16 - 32, XP055183762, ISSN: 1092-2172, DOI: 10.1128/MMBR.05015-11
- LISBOA ET AL.: "Nonsteroidal anti-inflammatory drugs may affect cytokine response and benefit healing of combat-related extremity wounds", SURGERY, vol. 161, no. 4, April 2017 (2017-04-01), pages 1164 - 73, XP055849798
- SABINDRA K. SAMAL, SAMAPIKA ROUTRAY, GANESH KUMAR VEERAMACHANENI, RUPESH DASH, MAHENDRAN BOTLAGUNTA: "Ketorolac salt is a newly discovered DDX3 inhibitor to treat oral cancer", SCIENTIFIC REPORTS, vol. 5, 28 April 2015 (2015-04-28), pages 9982, XP055318177, DOI: 10.1038/srep09982

## Description

### BACKGROUND OF THE DISCLOSURE

### Field of the Disclosure

This disclosure relates generally to compounds for use in methods of treatment This disclosure relates particularly to compositions for use in a method of treating cytokine storm and/or cytokine release syndrome.

### Description of Related Art

Cytokines are small proteins secreted by cells that play a central role in the control of immune and inflammatory responses (Tisoncik et al. Into the eye of the cytokine storm. Microbiol Mol Biol Rev. 2012;76(1):16-32). An overproduction of cytokines in response to infectious or noninfectious disease can result in what has been termed a "cytokine storm." For example, severe infections, such as in the lungs, caused by viral or bacterial agents, such as by cytomegalovirus, Epstein-Barr virus-associated hemophagocytic lymphohistiocytosis, group A streptococcus, influenza virus, variola virus, and severe acute respiratory syndrome coronavirus (SARS-CoV) local inflammation can spill over into the systemic circulation, producing systemic sepsis. Systemic sepsis is defined by persistent hypotension, hyper- or hypothermia, leukocytosis or leukopenia, and often thrombocytopenia (Levy MM, et al. 2003. 2001 SCCM/ESICM/ACCP/ATS/SIS International Sepsis Definitions Conference. Crit. Care Med. 31:1250-1256).

Cytokine release syndrome (CRS) is a related condition associated with CAR-T cell and antibody infusion immunotherapy (Liu et al., Journal of Hematology & Oncology (2018) 11:121). CRS has characteristic clinical presentations, including high fever, hypotension, hypoxia, and respiratory distress. Organ dysfunction including liver transaminitis and renal insufficiency can also result from CRS. Severe CRS complications can be life threatening and frequently require intensive care with vasopressors and/or ventilation support (Liu *et al.,* 2018). Other symptoms observed in CRS patients include appetite loss, nausea and vomiting, and diarrhea. (Kandola, Medical News Today (June 11, 2020)). Body weight loss in clinical conditions affects about 1% of the population, and there is currently no effective approach to address it. Progressive body weight loss in diseases such as chronic infection and cancer is a very common medical problem that can lead to overall health deterioration and death.

Pharmaceutical intervention to counter cytokine storm and/or CRS in afflicted patients focuses on the administration of immunomodulatory agents. Examples of such immunomodulatory agents include COX inhibitors (e.g., mesalamine or celecoxib), sphingosine receptor agonists (e.g., PF-04178903), anti-TNF agents, statins (e.g., simvastatin), OX40, and PPAR agonists (e.g., Gemfibrozil, pioglitazone, rosiglitazone, 15d-PGJ2, ciglitazone, and troglitazone). NSAID administration to patients who developed cytokine syndrome release following CAR-T therapy allowed to manage high fever and hypotension (Mi Jian-Qing et al., Blood, 2017, vol:130, page 3115).
However, additional therapies are required to further expand the armamentarium of therapeutic interventions available for treating cytokine storm and CRS.

### SUMMARY OF THE DISCLOSURE

The present invention is defined by the claims. Any subject-matter falling outside the scope of the claims is provided for information only.

Methods of treatment of the human body by therapy, referred to in this description, are not part of the present invention as such, but are described here in relation to compounds and pharmaceutical compositions for use in said methods for treatment of the human body by therapy, according to the present invention.

In a first aspect, the disclosure provides a therapeutically effective amount of ketorolac for use in a method of treating cytokine storm and/or cytokine release syndrome (CRS) in a subject in need thereof, wherein the ketorolac is (R)-ketorolac or racemic ketorolac, the method comprising: a) administering a therapeutically effective amount of ketorolac to the subject; and b) reducing a blood serum concentration of at least one of IL-1β, TNF-α, IL-6, IL-7, IL-10, MCP-1, MIP-1A, LIF, G-CSF, and GM-CSF in the subject.

In one embodiment, the cytokine storm and/or CRS is secondary to at least one of a viral infection, bacterial infection, cancer, and/or cancer treatment. In one embodiment of the first aspect, the viral infection is caused by one or more of a coronavirus, influenza, Epstein-Barr virus, respiratory syncytial virus, dengue virus, zika virus, west Nile virus, variola virus, cytomegalovirus, and HIV.

In one embodiment, the bacterial infection is caused by group A streptococcus.

In one embodiment, the cancer is an advanced stage cancer.

In one embodiment, the cancer is pancreatic cancer, lung cancer, or colorectal cancer.

In one embodiment, the cancer treatment comprises at least one of chemotherapy, radiotherapy, and immunotherapy.

In one embodiment, the immunotherapy comprises antibody therapy or chimeric antigen receptor (CAR) T cell treatment.

In one embodiment according to any of the preceding aspects or embodiments, the therapeutically effective amount of (R)-ketorolac or racemic ketorolac is about 1 mg to about 5 mg, about 5 mg to about 1000 mg, about 5 mg to about 800 mg, about 5 mg to about 500 mg, about 5 mg to about 200 mg, about 5 mg to about 100 mg, about 5 mg to about 50 mg, about 5 mg to about 25 mg, about 10 mg to about 1000 mg, about 10 mg to about 800 mg, about 10 mg to about 500 mg, about 10 mg to about 200 mg, about 10 mg to about 100 mg, or about 10 mg to about 50 mg.

In one embodiment according to any of the preceding aspects or embodiments, the amount of (R)-ketorolac or racemic ketorolac is selected based on the subject's disease severity, blood serum concentration of at least one of IL-1β, TNF-α, IL-6, IL-7, IL-10, MCP-1, MIP-1A, LIF, G-CSF, and GM-CSF, renal function, weight, age, and/or sex.

In a second aspect, not falling within the scope of the claimed subject-matter, the disclosure provides a pharmaceutical composition, comprising a therapeutically effective amount of enantiomerically pure (R)-ketorolac; and one or more excipients.

In one embodiment, the one or more excipients comprises a diluent, a lubricant, a solubilizer, an alcohol, a binder, a controlled release polymer, an enteric polymer, a disintegrant, a colorant, a flavorant, a sweetener, an antioxidant, a preservative, a pigment, an additive, a filler, a suspension agent, or surfactant.

In one embodiment, the pharmaceutical composition is a pharmaceutical dosage form.

In one embodiment, the pharmaceutical dosage form is a solid pharmaceutical dosage form.

In one embodiment, the pharmaceutical dosage form is an oral pharmaceutical dosage form.

In one embodiment, the oral pharmaceutical dosage form comprises a tablet, a capsule, a granule, a pellet, or a sachet.

In a third aspect, not falling within the scope of the claimed subject-matter, the disclosure provides a package comprising a pharmaceutical dosage form comprising a therapeutically effective amount of enantiomerically pure (R)-ketorolac (or racemic or (S)-ketorolac) and one or more excipients.

In a fourth aspect, the disclosure provides a method of treating cytokine storm and/or cytokine release syndrome (CRS) in a subject in need thereof, comprising: a) administering a therapeutically effective amount of racemic ketorolac to the subject; and b) reducing a blood serum concentration of at least one of IL-1(3, TNF-α, IL-6, IL-7, IL-10, MCP-1, MIP-1A, LIF, G-CSF, and GM-CSF in the subject.

In a fifth aspect, the disclosure provides a method of preventing or treating body weight loss associated with cytokine storm and/or cytokine release syndrome (CRS) in a subject in need thereof, comprising: a) administering a therapeutically effective amount of (R)-ketorolac or racemic ketorolac to the subject; and b) reducing body weight loss in the subject.

In a sixth aspect, the disclosure provides a method of preventing or treating diarrhea associated with cytokine storm and/or cytokine release syndrome (CRS) in a subject in need thereof, comprising: a) administering a therapeutically effective amount of racemic ketorolac or (R)-ketorolac to the subject; and b) reducing diarrhea in the subject.

In one embodiment according to either the fifth or sixth aspect, the therapeutically effective amount of ketorolac is about 1 mg to about 5 mg, about 5 mg to about 1000 mg, about 5 mg to about 800 mg, about 5 mg to about 500 mg, about 5 mg to about 200 mg, about 5 mg to about 100 mg, about 5 mg to about 50 mg, about 5 mg to about 25 mg, about 10 mg to about 1000 mg, about 10 mg to about 800 mg, about 10 mg to about 500 mg, about 10 mg to about 200 mg, about 10 mg to about 100 mg, or about 10 mg to about 50 mg.

In a seventh aspect, not falling within the scope of the claimed subject-matter, J the disclosure provides a pharmaceutical composition, comprising a therapeutically effective amount of racemic ketorolac; and one or more excipients.

### BRIEF DESCRIPTION OF DRAWINGS

FIG 1: Treatment effect of (R)-ketorolac on Cytokine Release Syndrome (CRS) ***in*** vivo. Serum concentrations (pg/mL) of interleukin 1 beta (IL-1β), tumor necrosis factor alpha (TNF-α), interleukin 6 (IL-6), interleukin 7 (IL-7), interleukin 10 (IL-10), monocyte chemoattractant protein-1 (MCP-1), macrophage inflammatory protein 1A (MIP-1A), leukemia inhibitory factor (LIF), granulocyte colony-stimulating factor (G-CSF), and granulocyte-macrophage colony-stimulating factor (GM-CSF) were determined in Balb/c mice treated with (R)-ketorolac compared to control.
FIG 2: Treatment effect of ketorolac on IL-6 serum levels in lipopolysaccharide (LPS)-induced CRS subjects *in vivo.* Serum concentrations (pg/mL) of IL-6 were determined in Balb/c mice treated with ketorolac compared to control. Asterisk indicates p value of <0.05.
FIG 3: Treatment effect of (R)-ketorolac on body weight loss caused by CRS *in **vivo**.* Body weight of mice with LPS-induced CRS was monitored after treatment with (R)-ketorolac, aspirin, or phosphate buffered saline (PBS). Asterisk indicates p value of <0.05.
FIG 4: Treatment effect of ketorolac on diarrhea caused by CRS *in vivo.* Mice with lipopolysaccharide (LPS)-induced CRS were monitored for diarrhea symptoms after treatment with (R)-ketorolac or PBS. Arrows indicate watery stool.

### DETAILED DESCRIPTION

Before describing the methods and compositions of the disclosure in detail, a number of terms will be defined. As used herein, the singular forms "a," "an," and "the" include plural referents unless the context clearly dictates otherwise. For example, reference to "a metabolite" means one or more metabolites.

It is noted that terms like "preferably," "commonly," and "typically" are not utilized herein to limit the scope of the methods and compositions as described herein or to imply that certain features are critical, essential, or even important to the structure or function of the claimed invention.

As used herein, the terms "or" and "and/or" are utilized to describe multiple components in combination or exclusive of one another. For example, "x, y, and/or z" can refer to "x" alone, "y" alone, "z" alone, "x, y, and z," "(x and y) or z," "x or (y and z)," or "x or y or z."

As used herein, the term "cytokine" refers to proteins secreted by cells that play a central role in the control of immune and inflammatory responses. Non-limiting examples of cytokines refered to herein include IL-1β, TNF-α, IL-6, IL-7, IL-10, MCP-1, MIP-1A, LIF, G-CSF, and GM-CSF.

As used herein, the term "cytokine storm" refers to an overproduction of cytokines in response to an infectious or a noninfectious disease, where the cytokines are released systemically, e.g., into the circulatory system of an afflicted individual.

As used herein, the term "cytokine release syndrome" or "CRS" refers to an overproduction of cytokines in response to CAR-T cell and/or antibody infusion immunotherapy, for example, as a cancer treatment.

As used herein, the term "cancer" refers to single cancerous cells as well as cancerous tissues. Non-limiting examples of cancers include pancreatic cancer, lung cancer, or colorectal cancer.

As used herein, the term "cancer treatment" refers to chemotherapy, radiotherapy, and immunotherapy.

As used herein, the term "advanced cancer" refers to metastatic and/or late stage cancer.

As used herein, the term "viral infection" refers to an infection of an individual with one or more viruses, such as a coronavirus, influenza, Epstein-Barr virus, respiratory syncytial virus, dengue virus, zika virus, west Nile virus, variola virus, cytomegalovirus, and human immunodeficiency virus (HIV).

As used herein, the term "bacterial infection" refers to an infection of an individual with one or more bacteria including, for example, group A streptococcus.

As used herein, the term "enantiomerically pure" when used in the context of describing a chemical compound refers to the chemical compound being present as a single ennatiomer, such as an S enantiomer or an R enantiomer. Conversely, a racemic chemical compound refers to the chemical compound being present as both the S and R-enantiomers. As used herein, enantiomerically pure compounds can include greater than about 60%, or greater than about 70%, or greater than about 80%, or greater than about 90% of one enantiomer over the other enantiomer.

As used herein, the terms "therapeutic amount," "therapeutically effective amount," and "effective amount" can be used interchangeably and refer to an amount of a compound that becomes available through the appropriate route of administration to treat a patient for a disorder, a condition, or a disease.

As used herein, the terms "treating," "treat," or "treatment" refer to either preventing, providing symptomatic relief, or curing a patient's disorder, condition, or disease.

As used herein, the terms "patient" and/or "subject" and/or "individual" can be used interchangeably and refer to an animal. For example, the patient, subject, or individual can be a mammal, such as a human.

As used herein, the terms "disorder," "condition," or "disease" refer to cytokine storm, CRS, or other pathologically elevated levels of cytokines.

### OVERVIEW

Provided herein is the use of the compound ketorolac and its enantiomers, and salts, solvates for modulation of expression of multiple inflammatory cytokines associated with cytokine storm and/or cytokine release syndrome (CRS). In particular, the present disclosure is directed to use of (R)-ketorolac for treating cytokine storm, CRS, or other pathologically elevated levels of cytokines caused by a disease state, as well as both weight loss and diarrhea. Moreover, it is further contemplated that racemic ketorolac, can also be used to combat cytokine storm and/or CRS as well as weight loss and diarrhea.

The present disclosure provides for the use of racemic or (R)-ketorolac in modulating cytokine levels and preventing or treating various disease states. In some embodiments, it is contemplated that racemic or (R)-ketorolac can each be substituted for the other to address each of the conditions disclosed herein. In other embodiments, one enantiomer is preferred over the other and the racemic mixture. For example, in certain embodiments (R)-ketorolac is preferred.

Accordingly, the present disclosure is directed to methods of treatment of cytokine storm and/or CRS in patients caused by, for example, the following disease states:
1) viral infections, including but not limited to, coronaviruses, influenza viruses, respiratory syncytial virus, dengue virus, zika virus, West Nile virus, and HIV;
2) advanced cancers including but not limited to pancreatic cancer, lung cancer, colorectal cancer; and
3) cancer patients under treatments including but limited to chemotherapy, antibody therapies and CAR-T cells.

Ketorolac is a nonsteroidal anti-inflammatory drug (NSAID) sold in the U.S. under the brand name Toradol^{®}. In this regard, ketorolac is believed to act by non-selective enzyme cyclooxygenase (COX) inhibition. In the present disclosure, enantiomerically pure (R)-ketorolac is shown to be effective in combating cytokine storm and/or CRS. However, it is further contemplated that derivatives (e.g., ketorolac tromethamine, galactosylated ketorolac, a ketorolac alkyl ester, a ketorolac piperazinylalkyl ester, and a ketorolac amide), pharmaceutically acceptable salts, solvates, hydrates, stereoisomers, clathrates, or prodrugs of (R)-ketorolac could also be used. Derivatives and prodrugs of Ketorolac do not fall within the scope of the claimed subject-matter.

### THERAPEUTIC METHODS

One aspect of the present disclosure is a method of treating cytokine storm and/or cytokine release syndrome (CRS) in a subject. The method includes administering a therapeutically effective amount of (R)-ketorolac or racemic ketorolac to the subject and reducing a blood serum concentration of at least one of IL-1β, TNF-α, IL-6, IL-7, IL-10, MCP-1, MIP-1A, LIF, G-CSF, and GM-CSF in the subject.

In some embodiments, administration of (R)-ketorolac or racemic ketorolac improves survival rate of subjects who experience cytokine storm or cytokine release syndrome (CRS), as described below. In certain embodiments, survival rate in subjects is improved in the range of about 10% to about 90% compared to untreated subjects who experience cytokine storm or cytokine release syndrome CRS. In some embodiments, survival rate is improved in the range of about 20% to about 80%. In some embodiments, survival rate is improved in the range of about 30% to about 70%. In some embodiments, survival rate is improved in the range of about 40% to about 60%. In some embodiments, survival rate in subjects is improved by about 50%.

In certain embodiments, (R)-ketorolac or racemic ketorolac is administered to improve survival rate at a dosage in the range of about 1 mg to about 1000 mg per kg body weight, e.g., in the range of about 5 mg to about 800 mg per kg body weight, or in the range of about 5 mg to about 500 mg per kg body weight, or in the range of about 5 mg to about 200 mg per kg body weight, or in the range of about 5 mg to about 100 mg per kg body weight, or in the range of about 5 mg to about 50 mg per kg body weight, or in the range of about 10 mg to about 1000 mg per kg body weight, or in the range of about 10 mg to about 800 mg per kg body weight, or in the range of about 10 mg to about 500 mg per kg body weight, or in the range of about 10 mg to about 200 mg per kg body weight, or in the range of about 10 mg to about 100 mg per kg body weight, or in the range of about 10 mg to about 50 mg per kg body weight, or in any 5 mg range therein, such as about 1 to about 5 mg per kg body weight or about 6 mg to about 10 mg per kg body weight or about 11 mg to about 15 mg per kg body weight, and so on.

In certain embodiments, (R)-ketorolac or racemic ketorolac is administered to the subject in a single dose to improve survival rate. In other embodiments, (R)-ketorolac or racemic ketorolac is administered to the subject in multiple doses to improve survival rate. In some embodiments, (R)-ketorolac or racemic ketorolac is administered to the subject daily for 6 days to improve survival rate.

In some embodiments, (R)-ketorolac or racemic ketorolac is administered to the subject orally to improve survival rate. In some embodiments, (R)-ketorolac or racemic ketorolac is administered to the subject through injection to improve survival rate.

In some embodiments of the present disclosure, the method includes administering a therapeutically effective amount of (R)-ketorolac or racemic ketorolac to the subject and reducing and/or preventing one or more symptoms related to or associated with CRS. In some embodiments, the symptoms are one or more of body weight loss and diarrhea. In some embodiments, the symptoms are reduced in one or more of occurrence and severity.

Another aspect of the present disclosure is a method of preventing or treating body weight loss in a subject in need thereof. In one aspect, the disclosure provides a method of preventing or treating body weight loss in a subject in need thereof, comprising: a) administering a therapeutically effective amount of (R)-ketorolac to the subject; and b) reducing body weight loss in the subject. In some aspects, the body weight loss in the subject is associated with cytokine storm and/or cytokine release syndrome. For example, in one aspect of the present disclosure is a method of preventing or treating body weight loss associated with cytokine storm and/or cytokine release syndrome (CRS) in a subject in need thereof, comprising administering a therapeutically effective amount of (R)-ketorolac or racemic ketorolac to the subject and reducing body weight loss in the subject. In certain embodiments, body weight loss in subjects is reduced in the range of about 10% to about 90%. In some embodiments, body weight loss is reduced in the range of about 20% to about 80%. In some embodiments, body weight loss is reduced in the range of about 30% to about 70%. In some embodiments, body weight loss is reduced in the range of about 40% to about 60%. In some embodiments, body weight loss in subjects is reduced by about 40%.

In certain embodiments, (R)-ketorolac or racemic ketorolac is administered to prevent or treat body weight loss at a dosage in the range of about 1 mg to about 1000 mg per kg body weight, e.g., in the range of about 5 mg to about 800 mg per kg body weight, or in the range of about 5 mg to about 500 mg per kg body weight, or in the range of about 5 mg to about 200 mg per kg body weight, or in the range of about 5 mg to about 100 mg per kg body weight, or in the range of about 5 mg to about 50 mg per kg body weight, or in the range of about 10 mg to about 1000 mg per kg body weight, or in the range of about 10 mg to about 800 mg per kg body weight, or in the range of about 10 mg to about 500 mg per kg body weight, or in the range of about 10 mg to about 200 mg per kg body weight, or in the range of about 10 mg to about 100 mg per kg body weight, or in the range of about 10 mg to about 50 mg per kg body weight, or in any 5 mg range therein, such as about 1 to about 5 mg per kg body weight or about 6 mg to about 10 mg per kg body weight or about 11 mg to about 15 mg per kg body weight, and so on.

In certain embodiments, (R)-ketorolac or racemic ketorolac is administered to the subject in a single dose to prevent or treat body weight loss. In other embodiments, (R)-ketorolac (or racemic or (S)-ketorolac) is administered to the subject in multiple doses to prevent or treat body weight loss.

In some embodiments, (R)-ketorolac or racemic ketorolac is administered to the subject orally to prevent or treat body weight loss. In some embodiments, (R)-ketorolac or racemic ketorolac is administered to the subject through injection to prevent or treat body weight loss.

Yet another aspect of the present disclosure is a method of preventing or treating diarrhea in a subject in need thereof. In one aspect, the disclosure provides a method of preventing or treating diarrhea in a subject in need thereof, comprising: a) administering a therapeutically effective amount of ketorolac to the subject; and b) reducing diarrhea in the subject. In some aspects, the body weight loss in the subject is associated with cytokine storm and/or cytokine release syndrome. For example, another aspect of the present is a method of preventing or treating diarrhea associated with cytokine storm and/or cytokine release syndrome (CRS) in a subject in need thereof, comprising: a) administering a therapeutically effective amount of ketorolac to the subject; and b) reducing diarrhea in the subject. In certain embodiments, acute diarrhea in subjects is reduced in the range of about 10% to about 90%. In some embodiments, acute diarrhea is reduced in the range of about 20% to about 80%. In some embodiments, acute diarrhea is reduced in the range of about 30% to about 70%. In some embodiments, acute diarrhea is reduced in the range of about 40% to about 70%. In some embodiments, acute diarrhea is reduced in the range of about 50% to about 70%. In some embodiments, acute diarrhea in subjects is reduced by about 65%.

In certain embodiments, racemic ketorolac or (R)-ketorolac is administered to prevent or treat diarrhea at a dosage in the range of about 1 mg to about 1000 mg per kg body weight, e.g., in the range of about 5 mg to about 800 mg per kg body weight, or in the range of about 5 mg to about 500 mg per kg body weight, or in the range of about 5 mg to about 200 mg per kg body weight, or in the range of about 5 mg to about 100 mg per kg body weight, or in the range of about 5 mg to about 50 mg per kg body weight, or in the range of about 10 mg to about 1000 mg per kg body weight, or in the range of about 10 mg to about 800 mg per kg body weight, or in the range of about 10 mg to about 500 mg per kg body weight, or in the range of about 10 mg to about 200 mg per kg body weight, or in the range of about 10 mg to about 100 mg per kg body weight, or in the range of about 10 mg to about 50 mg per kg body weight, or in any 5 mg range therein, such as about 1 to about 5 mg per kg body weight or about 6 mg to about 10 mg per kg body weight or about 11 mg to about 15 mg per kg body weight, and so on.

In certain embodiments, racemic ketorolac or (R)-ketorolac is administered to the subject in a single dose to prevent or treat diarrhea. In other embodiments, racemic ketorolac or (R)-ketorolac is administered to the subject in multiple doses to prevent or treat diarrhea.

In some embodiments, racemic ketorolac or (R)-ketorolac is administered to the subject orally to prevent or treat diarrhea. In some embodiments, racemic ketorolac or (R)-ketorolac is administered to the subject through injection to prevent or treat diarrhea.

As contemplated herein, the therapeutic compound (R)-ketorolac or racemic ketorolac can be provided in any desirable form, e.g., in any salt, crystalline, solvate and/or hydrate form.

Based on the disclosure herein, the person of ordinary skill in the art will select an appropriate dosage regimen for the administration of the therapeutic compound.

The person of ordinary skill in the art, based on the disclosure herein, can determine appropriate dosages of (R)-ketorolac or racemic ketorolac For example, in certain embodiments of the methods as otherwise described herein, (R)-ketorolac or racemic ketorolac can be provided at a dosage in the range of about 1 mg to about 200 mg, for example, about 3 mg to about 50 mg, about 6 mg to about 50 mg, or about 10 mg to about 25 mg per administration. However, in certain embodiments, no more than about 300 mg, or no more than about 200 mg, no more than about 100 mg, no more than about 75 mg, no more than about 50 mg, no more than about 25 mg, no more than about 10 mg, no more than about 6 mg, or no more than about 3 mg, or no more than about 1 mg of (R)-ketorolac or racemic ketorolac is administered to a subject in any 24 hour period.

In certain embodiments, (R)-ketorolac or racemic ketorolac is provided and/or administered at a dosage in the range of about 1 mg to about 1000 mg, *e.g*., in the range of about 5 mg to about 800 mg, or in the range of about 5 mg to about 500 mg, or in the range of about 5 mg to about 200 mg, or in the range of about 5 mg to about 100 mg, or in the range of about 5 mg to about 50 mg, or in the range of about 10 mg to about 1000 mg, or in the range of about 10 mg to about 800 mg, or in the range of about 10 mg to about 500 mg, or in the range of about 10 mg to about 200 mg, or in the range of about 10 mg to about 100 mg, or in the range of about 10 mg to about 50 mg, or in any 5 mg range therein, such as about 1 to about 5 mg or about 6 mg to about 10 mg, and so on.

For example, in one embodiment, all doses described herein can be provided at 1%, 2%, 3%, 4%, or 5%, or 10%, or 20%, or 30%, or 40%, or 50% the disclosed amount *(e.g.,* 500 mg TID can be 5 mg, 25 mg, 15 mg, 20 mg, 25 mg, 50 mg, 150 mg, 200 mg, or 250 mg TID).

As a general matter, suitable dosage amounts and dosing regimens may be selected in accordance with a variety of factors, including one or more of the subject's particular viral infection, bacterial infection, cancer, severity of the cancer, and/or cancer treatment, the genetic profile, age, health, sex, diet, and/or weight of the subject, the route of administration alone or in combination with pharmacological considerations including the activity, efficacy, bioavailability, pharmacokinetic, and toxicological profiles of the particular compound employed, whether a drug delivery system is utilized and whether the drug is administered as part of a drug combination. Therefore, the dosing regimen to be employed may vary widely and may necessarily deviate from the dosage regimens set forth herein.

In another embodiment, the duration of administration of the therapeutic compound can be determined by indication of treatment effectiveness. For example, the therapeutic compound can be administered for a period of time during which a decrease in a marker is observed. For example, in one particular embodiment, administration of the therapeutic compound continues while blood levels of proinflammatory cytokines decrease to a normal level.

### PHARMACEUTICAL COMPOSITIONS AND DOSAGE FORMS

The contemplated pharmaceutical compositions, not falling within the scope of the claimed subject-matter, can include (R)-ketorolac (or racemic or (S)-ketorolac) or pharmaceutically acceptable salts or derivatives thereof alone or together with one or more pharmaceutically acceptable carriers therefor.

The compositions of the present disclosure can include one or more of (R)-, (S)-, and racemic mixtures of ketorolac in combination with other active ingredients. In some embodiments, the compositions can include ketorolac in combination with one or more immunomodulatory agents. For example, contemplated immunomodulatory agents can include COX inhibitors (e.g., mesalamine or celecoxib), sphingosine receptor agonists (e.g., PF-04178903), anti-TNF agents, statins (e.g., simvastatin), OX40, and PPAR agonists (e.g., Gemfibrozil, pioglitazone, rosiglitazone, 15d-PGJ2, ciglitazone, and troglitazone). The compositions including ketorolac and other active ingredients can be used for treating the various disease states and conditions disclosed herein.

The pharmaceutical composition can further include an amount of one or more excipients, and a ratio of the therapeutically effective amount of (R)-ketorolac (or racemic or (S)-ketorolac) to the amount of the one or more excipients (weight:weight) is about 1:0.1 to about 1:40 or about 1:1 to about 1:10. The excipients can include a diluent, a lubricant, a solubilizer, an alcohol, a binder, a controlled release polymer, an enteric polymer, a disintegrant, a colorant, a flavorant, a sweetener, an antioxidant, a preservative, a pigment, an additive, a filler, a suspension agent, or surfactant.

Depending on the dosing regimen, each administration of a pharmaceutical composition including (R)-ketorolac (or racemic or (S)-ketorolac) can be performed, for example, by giving a patient a single unit dose *(e.g.,* pill, capsule, other oral dosage form) or injection, multiple unit doses or injections, or continuously (e.g., intravenous or slow-release patch), or otherwise as disclosed herein. For example, a pharmaceutical composition including (R)-ketorolac (or racemic or (S)-ketorolac) can be formulated for oral, transdermal, intraspinal, intrathecal, inhalation, subcutaneous, intravenous, intramuscular, or transmucosal administration, or for administration via osmotic pump, microcapsule, implant, or suspension. It is further contemplated that (R)-ketorolac (or racemic or (S)-ketorolac) can be formulated for different routes of administration at the same time or within the same administration.

In certain embodiments, it may be desirable to administer (R)-ketorolac (or racemic or (S)-ketorolac) using a time-release or slow-release formulation, or transdermally (e.g., using a patch) in order to provide an administration half-life of sufficient length. When (R)-ketorolac (or racemic or (S)-ketorolac) is administered transdermally or using a time-release or slow-release formulation, it is desirably released over a time period between two and twelve hours in duration; between two and six hours in duration; or between six and twelve hours in duration. In order to provide a high *in vivo* concentration of (R)-ketorolac (or racemic or (S)-ketorolac) in a short amount of time, it may be desirable to administer (R)-ketorolac (or racemic or (S)-ketorolac) using a rapidly absorbed loading dose. To provide a high *in vivo* concentration of (R)-ketorolac (or racemic or (S)-ketorolac) quickly as well as a sufficiently long administration half-life, it may be desirable to use both a rapidly absorbed loading dose and transdermal administration or a time-release or slow-release formulation. Examples of transdermal patches that can be used in the present disclosure include those disclosed in U.S. Patent Nos. 3,598,122, 3,598,123, 4,379,454, 4,286,592, 4,317,557, and 4,573,995. However, any dosage form is contemplated to allow an individually tailored dosing regimen of (R)-ketorolac (or racemic or (S)-ketorolac) to fit a specific person's needs.

In one particular embodiment, the pharmaceutical dosage form is a solid pharmaceutical dosage form.

Compositions containing the active pharmaceutical ingredients may also contain one or more inactive pharmaceutical excipients such as diluents, lubricants, solubilizers, alcohols, binders, controlled release polymers, enteric polymers, disintegrants, colorants, flavorants, sweeteners, antioxidants, preservatives, pigments, additives, fillers, suspension agents, surfactants (for example, anionic, cationic, amphoteric and nonionic), and the like. Various FDA-approved topical inactive ingredients are found at the FDA's "The Inactive Ingredients Database" that contains inactive ingredients specifically intended as such by the manufacturer, whereby inactive ingredients can also be considered active ingredients under certain circumstances, according to the definition of an active ingredient given in 21 CFR 210.3(b)(7). Alcohol is a good example of an ingredient that may be considered either active or inactive depending on the product formulation.

Numerous dosage forms are contemplated herein including those suitable for oral, rectal, nasal, topical (including buccal and sublingual), transdermal, vaginal, injection/injectable, or parental (including subcutaneous, intramuscular, intravenous, and intradermal) administration, or inhalation or insufflation.

### KITS

As used herein, a kit, not falling within the scope of the claimed subject-matter, may be a packaged collection of related materials, including, for example, a plurality of packages as described above including a single and/or a plurality of dosage forms along with instructions for use.

In another embodiment, a kit includes a solution, suspension, patch, cream or other dosage form and instructions to apply the dosage form directly to the skin, nasal cavity, respiratory tract, eye, or ear by conventional means, for example with a dropper, pipette, spray, applicator, or wipe, or as otherwise disclosed herein.

The invention will be further described in the following examples, which do not limit the scope of the invention described in the claims.

### EXAMPLES

The Examples that follow are illustrative of specific embodiments of the invention, and various uses thereof. They are set forth for explanatory purposes only and are not taken as limiting the invention.

### EXAMPLE No. 1: Treatment of cancer-induced CRS with (R)-ketorolac

***Summary:*** the present example determined effectiveness of (R)-ketorolac against cytokine release syndrome *in vivo.*

### Study Protocol

### 1.1 Preparation of tumor cells

Cytokine storm inducing C26 tumor cells were cultured in Advanced RPMI 1640 medium supplied with 5% fetal bovine serum and 1% penicillin/streptomycin and maintained in a 5% CO₂, 37°C humidified incubator. Tumor cells with viability of >90% were chosen for the study. Viable tumor cells (1 x 10⁶ cells) were re-suspended in 100 µL of PBS and subcutaneously injected into the left flank of mice. All procedures were performed in laminar flow hood following sterile techniques.

### 1.2 Disease model establishment

6-8 week BALB/c male and female mice were used for these studies. Following tumor inoculation, the animals were returned to the cage. The mice were monitored for the appearance of palpable tumor and cytokine storm development by observing health condition change and testing serum cytokines with Mouse-Cytokine-32-Plex assay.

### 1.3 Route and mode of administration of the test substance

After mice lost 10% body weight, test compound (R)-ketorolac or control vehicle (PBS) was administered by gavage 0.2 mg daily for six days. 2-4 mice were included in each experimental group. On day 7, mouse blood samples were collected and allowed to clot for one hour at room temperature. The samples were then centrifuged for 15 min at 3,000 rpm (1500 × g) at 4° C. Thirty microliters of serum per treatment animal were assessed for cytokine profiles using a Mouse-Cytokine-32-Plex assay (Eve Technologies Corporation, Calgary, AB, Canada).

### Results:

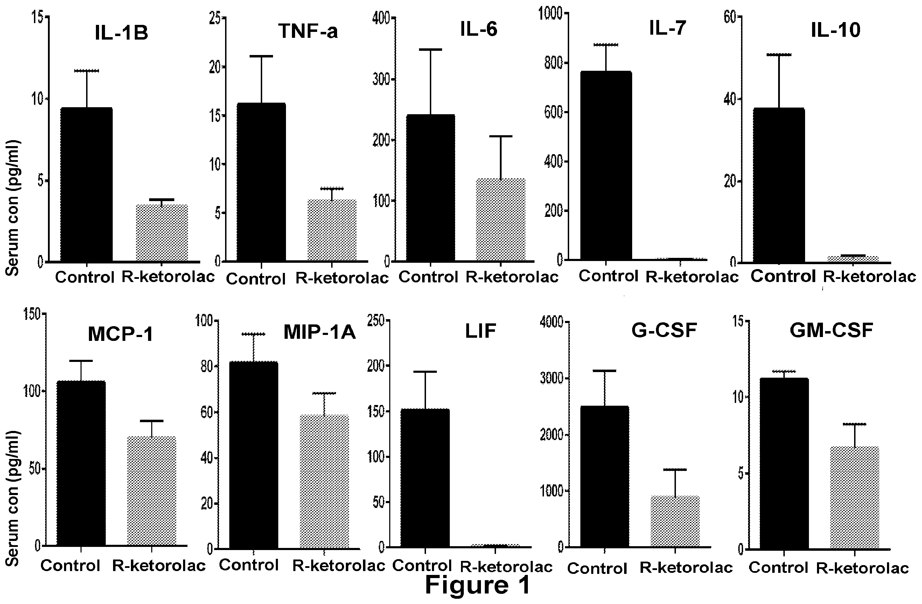

As shown in Figure 1, administration of oral (R)-ketorolac was effective in reducing serum concentrations of IL-1β, TNF-α, IL-6, IL-7, IL-10, MCP-1, MIP-1A, LIF, G-CSF, and GM-CSF compared to PBS controls in a tumor-induced cytokine storm model.

### Conclusion:

Enantiomerically pure, (R)-ketorolac represents a new therapy option for combating cytokine storm and/ or CRS.

### EXAMPLE No. 2: Testing survival upon treatment of lipopolysaccharide (LPS)-induced CRS with (R)-ketorolac

***Summary***: the present example determined effectiveness of (R)-ketorolac on survival of CRS *in vivo.*

### Study Protocol

### 1.1 Disease model establishment

8-week-old female BALB/c mice were used for these studies. Injection of LPS has previously been shown to induce a cytokine storm (Su et al., Biomedicine & Pharmacotherapy, Vol. 131, November 2020). The mice were challenged with 0.2 mg LPS (Sigma Cat. No. L2630) dissolved in 0.1 mL PBS via intraperitoneal injection. The mice were monitored for health condition and survival for a minimum of seven days.

### 1.2 Route and mode of administration of the test substance

Within 2 minutes of the LPS injection, test compound (R)-ketorolac or control vehicle (PBS) was administered at 10 mg per kg body weight by single intraperitoneal injection. 3 mice were included per experimental group.

### Results:

As shown in Table 1, administration of oral (R)-ketorolac resulted in increased survival versus administration of PBS. In the control group, a 33.3% survival rate was observed (4 of 6 mice died). However, treatment with 10 mg/kg oral (R)-ketorolac increased the survival rate to 83.3% (only 1 in 6 mice died), which illustrates an increase in survival rate of 50%.

**Table 1. Survival of Control and (R)-ketorolac-treated mice with LPS-induced CRS**

| Group | LPS challenge | Treatment | Survival rate |
|---|---|---|---|
| Control n=6 | Mice were challenged with 0.2mg LPS via intraperitoneal injection | PBS | 2/6 |
| R-Ketorolac n=6 | | R-Ketorolac | 5/6 |

### Conclusion:

Administration of (R)-ketorolac is a viable therapeutic approach to rescue subjects from death caused by CRS. Early administration of R-ketorolac (to prevent the cytokine level from elevating substantially) can be a particularly effective approach.

### EXAMPLE No. 3: Testing IL-6 levels upon treatment of lipopolysaccharide (LPS)-induced CRS with ketorolac

***Summary:*** the present example determined the effect of ketorolac on IL-6 levels of subjects with CRS *in vivo.*

### Study Protocol

### 1.1 Disease model establishment

8-week-old male BALB/c mice were used for these studies. The mice were challenged with 50 µg LPS (Sigma Cat. No. L2630) dissolved in 0.1 mL PBS, a dosage designed to ensure all animals survived the challenge, via intraperitoneal injection. Mouse serum was collected 2 hours after the challenge, and IL-6 was measured with the Mouse Il-6 Flex Set (BD Biosciences Cat. No. 558301).

### 1.2 Route and mode of administration of the test substance

Within 2 minutes of the LPS injection, test compound ketorolac (racemic mixture) or control vehicle (PBS) was administered at 10 mg per kg body weight by single intraperitoneal injection. 5 mice were included per experimental group.

### Results:

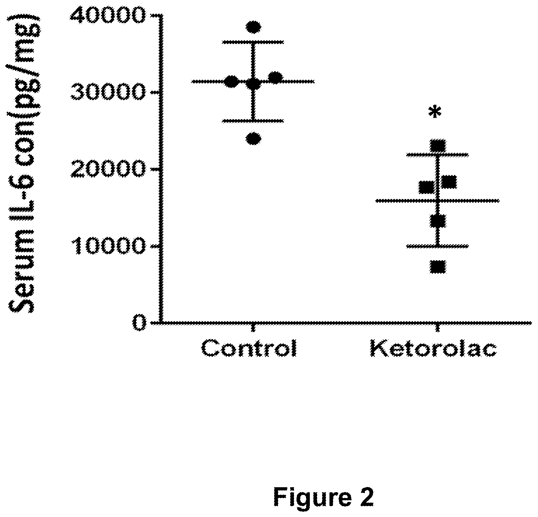

As shown in Figure 2, administration of ketorolac resulted in decreased serum IL-6 levels versus administration of PBS. p=0.0023.

### Conclusion:

Administration of ketorolac is a viable therapeutic approach to reduce elevated cytokine levels caused by CRS.

### EXAMPLE No. 4: (R)-ketorolac Treatment of weight loss caused by low-dose lipopolysaccharide (LPS)-induced CRS

***Summary***: the present example determined effectiveness of (R)-ketorolac on loss of body weight due to CRS *in* vivo.

### Study Protocol

### 1.1 Disease model establishment

8-week-old female BALB/c mice were used for these studies. The mice were challenged with 0.05 mg LPS (Sigma Cat. No. L2630) via intraperitoneal injection. Body weight was measured before LPS challenge and the next day after the challenge.

### 1.2 Route and mode of administration of the test substance

Within 2 minutes of LPS injection, test compound (R)-ketorolac (0.3 mg, corresponding to 15 mg/kg), aspirin (0.3 mg, corresponding to 15 mg/kg), or control vehicle (PBS) was orally administered.

### Results:

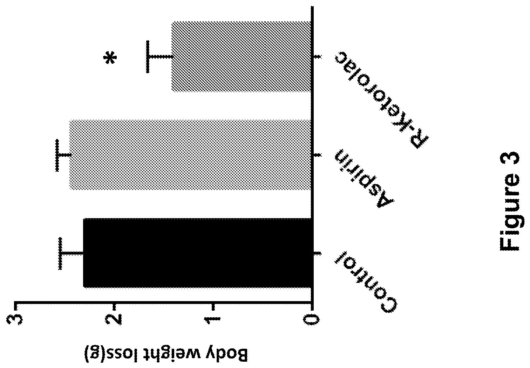

As shown in Figure 3, compared with aspirin, (R)-ketorolac treatment significantly reduced body weight loss induced by low-dose LPS challenge. p=0.0267.

### Conclusion:

Administration of (R)-ketorolac can reduce weight loss caused by CRS. Unlike other nonsteroidal anti-inflanunatory drugs (NSAIDs), such as aspirin, (R)-ketorolac is a viable treatment approach to clinically manage clinical weight loss in the context of CRS and/or other conditions.

### EXAMPLE No. 5: Ketorolac treatment of acute diarrhea caused by high-dose lipopolysaccharide (LPS)-induced CRS

***Summary***: the present example determined effectiveness of ketorolac on acute diarrhea caused by CRS *in vivo.*

### Study Protocol

### 1.1 Disease model establishment

8-10-week-old female BALB/c mice were used for these studies. The mice were challenged with LPS at 15 mg per kg body weight, a dosage which had been empirically determined to induce diarrhea in 100% of tested subjects, via intraperitoneal injection. The mice were monitored for diarrhea symptoms, with diarrhea defined by the presence of watery stools 6 hours after treatment.

### 1.2 Route and mode of administration of the test substance

Within 2 minutes of the LPS injection, test compound ketorolac (racemic mixture, suspended in PBS) or control vehicle (PBS) was administered at 0.1 mg (corresponding to 5 mg/kg) by single intraperitoneal injection.

### Results:

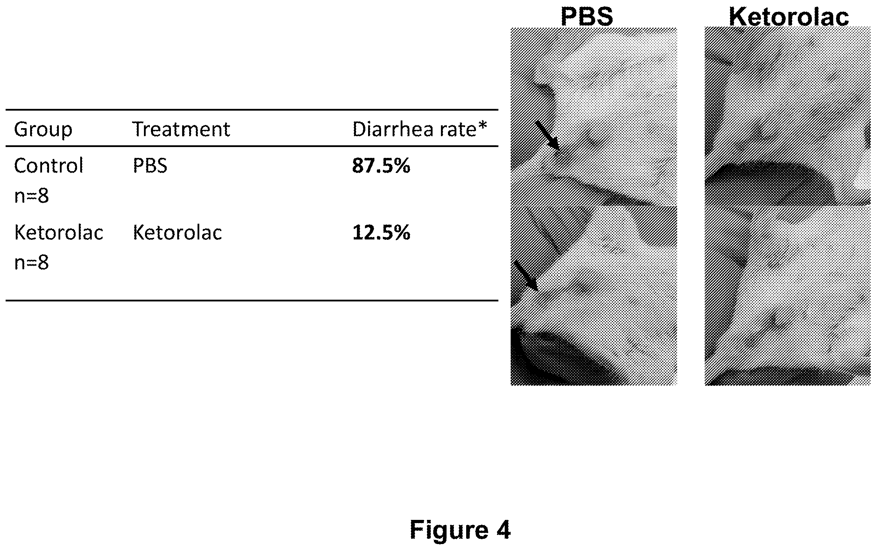

As shown in Figure 4, diarrhea was present in 87.5% of mice in the control group (n=8) and 12.5% of mice in the ketorolac group (n=8). Therefore, treatment with a 0.1 mg (corresponding to 5 mg/kg) intraperitoneal injection of ketorolac decreased acute diarrhea occurrence caused by high-dose LPS-induced CRS by 65%.

### Conclusion:

Administration of ketorolac can prevent acute diarrhea associated with CRS. The finding that ketorolac can treat diarrhea in the classic LPS model is surprising, as NSAIDs are generally considered to contribute to adverse gastrointestinal events. The finding is also clinically significant, as diarrhea is a severe symptom which frequently occurs during CRS. This symptom occurs in the context of many infectious diseases, including COVID-19. These results indicate that ketorolac is a very effective drug to treat diarrhea in disease conditions mediated by CRS and has broad clinical application. Furthermore, this approach is particularly promising with regard to rescuing patients in the acute care setting because (R)-ketorolac is not active against cyclooxygenases (COX1 and COX2), and thus will not cause events due to inhibition of these enzymes.

## Claims

1. A therapeutically effective amount of ketorolac for use in a method of treating cytokine storm and/or cytokine release syndrome (CRS) in a subject in need thereof, wherein the ketorolac is (R)-ketorolac or racemic ketorolac, the method comprising:
a) administering a therapeutically effective amount of ketorolac to the subject; and
b) reducing a blood serum concentration of at least one of IL-1β, TNF-α, IL-6, IL-7, IL-10, MCP-1, MIP-1A, LIF, G-CSF, and GM-CSF in the subject.

2. The therapeutically effective amount of ketorolac for use of claim 1, wherein the cytokine storm and/or CRS is secondary to at least one of a viral infection, bacterial infection, cancer, and/or cancer treatment; optionally wherein:
a) the viral infection is caused by one or more of a coronavirus, influenza, Epstein-Barr virus, respiratory syncytial virus, dengue virus, zika virus, west Nile virus, variola virus, cytomegalovirus, and HIV; or
b) the bacterial infection is caused by group A streptococcus; or
c) the cancer is an advanced stage cancer, optionally wherein the cancer is pancreatic cancer, lung cancer, or colorectal cancer; or
d) the cancer treatment comprises at least one of chemotherapy, radiotherapy, and immunotherapy, optionally wherein immunotherapy comprises antibody therapy or CAR T cell treatment.

3. The therapeutically effective amount of ketorolac for use of any of the preceding claims, wherein the therapeutically effective amount of (R)-ketorolac is about 1 mg to about 5 mg, about 5 mg to about 1000 mg, about 5 mg to about 800 mg, about 5 mg to about 500 mg, about 5 mg to about 200 mg, about 5 mg to about 100 mg, about 5 mg to about 50 mg, about 5 mg to about 25 mg, about 10 mg to about 1000 mg, about 10 mg to about 800 mg, about 10 mg to about 500 mg, about 10 mg to about 200 mg, about 10 mg to about 100 mg, or about 10 mg to about 50 mg.

4. The therapeutically effective amount of ketorolac for use of any of the preceding claims, wherein the amount of (R)-ketorolac (or racemic ketorolac) is selected based on the subject's disease severity, blood serum concentration of at least one of IL-1β, TNF-α, IL-6, IL-7, IL-10, MCP-1, MIP-1A, LIF, G-CSF, and GM-CSF, renal function, weight, age, and/or sex.

5. The therapeutically effective amount of ketorolac for use of claim 1, wherein the ketorolac is racemic.

6. A therapeutically effective amount of (R)-ketorolac (or racemic ketorolac) for use in a method of prevention or treatment of body weight loss or diarrhea associated with cytokine storm and/or cytokine release syndrome (CRS) in a subject in need thereof, the method comprising:
a) administering a therapeutically effective amount of (R)-ketorolac (or racemic ketorolac) to the subject; and
b) reducing occurrence or severity of body weight loss or diarrhea in the subject.

7. The therapeutically effective amount of (R)-ketorolac (or racemic ketorolac) for use in claim 6, wherein the therapeutically effective amount of ketorolac is about 1 mg to about 5 mg, about 5 mg to about 1000 mg, about 5 mg to about 800 mg, about 5 mg to about 500 mg, about 5 mg to about 200 mg, about 5 mg to about 100 mg, about 5 mg to about 50 mg, about 5 mg to about 25 mg, about 10 mg to about 1000 mg, about 10 mg to about 800 mg, about 10 mg to about 500 mg, about 10 mg to about 200 mg, about 10 mg to about 100 mg, or about 10 mg to about 50 mg.

## Patentansprüche

1. Therapeutisch wirksame Menge Ketorolac zur Verwendung in einem Verfahren zur Behandlung von Zytokinsturm und/oder Zytokin-Freisetzungssyndrom (cytokine release syndrome, CRS) in einem Subjekt, das dessen bedarf, wobei das Ketorolac (R)-Ketorolac oder racemisches Ketorolac ist, wobei das Verfahren Folgendes umfasst:
a) Verabreichen einer therapeutisch wirksamen Menge Ketorolac an das Subjekt; und
b) Verringern einer Blutserumkonzentration von mindestens einem aus IL-1β, TNF-α, IL-6, IL-7, IL-10, MCP-1, MIP-1A, LIF, G-CSF und GM-CSF in dem Subjekt.

2. Therapeutisch wirksame Menge Ketorolac zur Verwendung nach Anspruch 1, wobei der Zytokinsturm und/oder das CRS sekundär zu mindestens einem aus einer Virusinfektion, einer bakteriellen Infektion, Krebs und/oder Krebsbehandlung ist, wobei optional:
a) die Virusinfektion verursacht wird durch eines oder mehrere aus einem Coronavirus, Influenza, Epstein-Barr-Virus, respiratorischem Synzytial-Virus, Dengue-Virus, Zika-Virus, West-Nil-Virus, Variola-Virus, Zytomegalievirus und HIV; oder
b) die bakterielle Infektion verursacht wird durch Gruppe-A-Streptokokken; oder
c) der Krebs ein Krebs im fortgeschrittenen Stadium ist, wobei optional der Krebs Bauchspeicheldrüsenkrebs, Lungenkrebs oder Darmkrebs ist; oder
d) die Krebsbehandlung mindestens eines aus Chemotherapie, Strahlentherapie und Immuntherapie umfasst, wobei optional Immuntherapie Antikörpertherapie oder CAR-T-Zell-Behandlung umfasst.

3. Therapeutisch wirksame Menge Ketorolac zur Verwendung nach einem der vorhergehenden Ansprüche, wobei die therapeutisch wirksame Menge (R)-Ketorolac etwa 1 mg bis etwa 5 mg, etwa 5 mg bis etwa 1000 mg, etwa 5 mg bis etwa 800 mg, etwa 5 mg bis etwa 500 mg, etwa 5 mg bis etwa 200 mg, etwa 5 mg bis etwa 100 mg, etwa 5 mg bis etwa 50 mg, etwa 5 mg bis etwa 25 mg, etwa 10 mg bis etwa 1000 mg, etwa 10 mg bis etwa 800 mg, etwa 10 mg bis etwa 500 mg, etwa 10 mg bis etwa 200 mg, etwa 10 mg bis etwa 100 mg oder etwa 10 mg bis etwa 50 mg beträgt.

4. Therapeutisch wirksame Menge Ketorolac zur Verwendung nach einem der vorhergehenden Ansprüche, wobei die Menge (R)-Ketorolac (oder racemisches Ketorolac) ausgewählt ist basierend auf Krankheitsschwere, Blutserumkonzentration von mindestens einem aus IL-1β, TNF-α, IL-6, IL-7, IL-10, MCP-1, MIP-1A, LIF, G-CSF und GM-CSF, Nierenfunktion, Gewicht, Alter und/oder Geschlecht des Subjekts.

5. Therapeutisch wirksame Menge Ketorolac zur Verwendung nach Anspruch 1, wobei das Ketorolac racemisch ist.

6. Therapeutisch wirksame Menge (R)-Ketorolac (oder racemisches Ketorolac) zur Verwendung in einem Verfahren zur Prävention oder Behandlung von Körpergewichtsverlust oder Diarrhöe im Zusammenhang mit Zytokinsturm und/oder Zytokin-Freisetzungssyndrom (CRS) in einem Subjekt, das dessen bedarf, wobei das Verfahren Folgendes umfasst:
a) Verabreichen einer therapeutisch wirksamen Menge (R)-Ketorolac (oder racemisches Ketorolac) an das Subjekt; und
b) Verringern des Auftretens oder der Schwere von Körpergewichtsverlust oder Diarrhöe in dem Subjekt.

7. Therapeutisch wirksame Menge (R)-Ketorolac (oder racemisches Ketorolac) zur Verwendung nach Anspruch 6, wobei die therapeutisch wirksame Menge (R)-Ketorolac (oder racemisches Ketorolac) etwa 1 mg bis etwa 5 mg, etwa 5 mg bis etwa 1000 mg, etwa 5 mg bis etwa 800 mg, etwa 5 mg bis etwa 500 mg, etwa 5 mg bis etwa 200 mg, etwa 5 mg bis etwa 100 mg, etwa 5 mg bis etwa 50 mg, etwa 5 mg bis etwa 25 mg, etwa 10 mg bis etwa 1000 mg, etwa 10 mg bis etwa 800 mg, etwa 10 mg bis etwa 500 mg, etwa 10 mg bis etwa 200 mg, etwa 10 mg bis etwa 100 mg oder etwa 10 mg bis etwa 50 mg beträgt.

## Revendications

1. Quantité thérapeutiquement efficace de kétorolac destinée à être utilisée dans une méthode de traitement du choc cytokinique et/ou du syndrome de libération des cytokines (SLC) chez un sujet qui en a besoin, le kétorolac étant du (R)-kétorolac ou du kétorolac racémique, la méthode comprenant :
a) l'administration d'une quantité thérapeutiquement efficace de kétorolac au sujet, et
b) la réduction de la concentration sérique de IL-10, TNF-α, IL-6, IL-7, IL-10, MCP-1, MIP-1A, LIF, G-CSF et/ou GM-CSF chez le sujet.

2. Quantité thérapeutiquement efficace de kétorolac destinée à être utilisée selon la revendication 1, le choc cytokinique et/ou le SLC étant secondaires à au moins une affection parmi une in 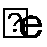 ction virale, une in 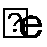 ction bactérienne et un cancer, et/ou un traitement anticancéreux; étant entendu éventuellement que :
a) l'in 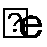 ction virale est due à un ou plusieurs des virus suivants : le coronavirus, la grippe, le virus d'Epstein-Barr, le virus respiratoire syncytial, le virus de la dengue, le virus Zika, le virus du Nil occidental, le virus de la variole, le cytomégalovirus et le VIH, ou
b) l'in 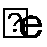 ction bactérienne est due à un streptocoque du groupe A, ou
c) le cancer est un cancer à un stade avancé et étant éventuellement un cancer du pancréas, un cancer du poumon ou un cancer colorectal, ou
d) le traitement anticancéreux comprend la chimiothérapie, la radiothérapie et/ou l'immunothérapie, laquelle immunothérapie comprend éventuellement une thérapie par anticorps ou un traitement par cellules CAR-T.

3. Quantité thérapeutiquement efficace de kétorolac destinée à être utilisée selon l'une quelconque des revendications précédentes, la quantité thérapeutiquement efficace de (R)-kétorolac étant d'environ 1 mg à environ 5 mg, d'environ 5 mg à environ 1000 mg, d'environ 5 mg à environ 800 mg, d'environ 5 mg à environ 500 mg, d'environ 5 mg à environ 200 mg, d'environ 5 mg à environ 100 mg, d'environ 5 mg à environ 50 mg, d'environ 5 mg à environ 25 mg, d'environ 10 mg à environ 1000 mg, d'environ 10 mg à environ 800 mg, d'environ 10 mg à environ 500 mg, d'environ 10 mg à environ 200 mg, d'environ 10 mg à environ 100 mg ou d'environ 10 mg à environ 50 mg.

4. Quantité thérapeutiquement efficace de kétorolac destinée à être utilisée selon l'une quelconque des revendications précédentes, la quantité de (R)-kétorolac (ou de kétorolac racémique) étant choisie compte tenu de la sévérité de la maladie du sujet, de la concentration sérique de IL-1β, TNF-α, IL-6, IL-7, IL-10, MCP-1, MIP-1A, **LIF,** G-CSF et GM-CSF, de la 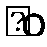 nction rénale, du poids, de l'âge et/ou du sexe.

5. Quantité thérapeutiquement efficace de kétorolac destinée à être utilisée selon la revendication 1, le kétorolac étant racémique.

6. Quantité thérapeutiquement efficace de (R)-kétorolac (ou de kétorolac racémique) destinée à être utilisée dans une méthode de prévention ou de traitement de la perte de poids corporel ou de la diarrhée associées au choc cytokine et/ou au syndrome de libération des cytokines (SLC) chez un sujet qui en a besoin, la méthode comprenant :
a) l'administration d'une quantité thérapeutiquement efficace de (R)-kétorolac (ou de kétorolac racémique) au sujet, et
b) la réduction de la survenue ou de la sévérité de la perte de poids corporel ou de la diarrhée chez le sujet.

7. Quantité thérapeutiquement efficace de (R)-kétorolac (ou de kétorolac racémique) destinée à être utilisée selon la revendication 6, la quantité thérapeutiquement efficace de kétorolac étant d'environ 1 mg à environ 5 mg, d'environ 5 mg à environ 1000 mg, d'environ 5 mg à environ 800 mg, d'environ 5 mg à environ 500 mg, d'environ 5 mg à environ 200 mg, d'environ 5 mg à environ 100 mg, d'environ 5 mg à environ 50 mg, d'environ 5 mg à environ 25 mg, d'environ 10 mg à environ 1000 mg, d'environ 10 mg à environ 800 mg, d'environ 10 mg à environ 500 mg, d'environ 10 mg à environ 200 mg, d'environ 10 mg à environ 100 mg, ou d'environ 10 mg à environ 50 mg.
